# EUROPEAN PATENT SPECIFICATION

(11) **EP 1 753 467 B1**
(45) Date of publication and mention of the grant of the patent: **09.01.2013**
(21) Application number: 05782465.8
(22) Date of filing: 13.05.2005
(51) Int. Cl.: A61K 51/04, A61K 101/00, A61K 101/02

(54) **DIAGNOSTIC AGENTS FOR POSITRON EMISSION IMAGING USING RADIOLABELED HALOGENATED XANTHENES AND METHODS FOR POSITRON EMISSION IMAGING WITH RADIOLABELED HALOGENATED XANTHENE DIAGNOSTIC AGENTS**
DIAGNOSEMITTEL FÜR POSITRONENEMISSIONSBILDGEBUNG UNTER VERWENDUNG VON RADIOAKTIV MARKIERTEN HALOGENIERTEN XANTHENEN UND VERFAHREN FÜR POSITRONENEMISSIONSBILDGEBUNG MIT RADIOAKTIV MARKIERTEN HALOGENIERTEN XANTHEN-DIAGNOSEMITTELN
AGENTS DIAGNOSTIQUES POUR L'IMAGERIE PAR EMISSION DE POSITRON UTILISANT DES XANTHENES HALOGENES RADIOMARQUES ET METHODES D'IMAGERIE PAR EMISSION DE POSITRON AVEC DES AGENTS DIAGNOSTIQUES DE TYPE XANTHENES HALOGENES RADIOMARQUES

(30) Priority: 10.06.2004 US 578622 P; 03.02.2005 US 49797; 03.02.2005 US 50512
(43) Date of publication of application: 21.02.2007
(73) Proprietor: Provectus Pharmatech, Inc., Knoxville, TN 37931 (US)
(72) Inventor: SCOTT, Timothy, C., Knoxville, TN 37932 (US); DEES, Craig, H., Knoxville, TN 37931 (US); WACHTER, Eric, A., Oak Ridge, TN 37830 (US)
(74) Representative: Hill, Christopher Michael
(86) International application number: PCT/US2005/016836
(87) International publication number: WO 2006/001925

(56) References cited:
- WO-A-01/72301
- GB-A- 846 674
- US-A- 4 256 727
- THAKUR ET AL: "Radioiodinated rhodamine-123: Preparationand preliminary evaluation as an agent for tumor scintigraphy" INTERNATIONAL JOURNAL OF RADIATION APPLICATIONS ANDINSTRUMENTATION PART B: NUCLEAR MEDICINE AND BIOLOGY, ELSEVIER SCIENCE PUBLISHERS, NEW YORK, NY, US, vol. 15, no. 5, 1 January 1988 (1988-01-01), pages 517-524, XP022416763 ISSN: 0883-2897
- SAHA G B ET AL: "CYCLOTRONS AND POSITRON EMISSION TOMOGRAPHY RADIOPHARMACEUTICALS FOR CLINICAL IMAGING" SEMINARS IN NUCLEAR MEDICINE, vol. 22, no. 3, 1992, pages 150-161, XP002490045 ISSN: 0001-2998
- SERAFINI A N ET AL: "Iodine-123-Rose Bengal: An improved hepatobiliary imaging agent" JOURNAL OF NUCLEAR MEDICINE, SOCIETY OF NUCLEAR MEDICINE, RESTON, VA, US, vol. 16, no. 7, 1 January 1975 (1975-01-01), pages 629-632, XP002453459 ISSN: 0161-5505

## Description

### BACKGROUND OF THE INVENTION

The present invention is directed to new diagnostic agents, and methods of use of such agents, for medical imaging using positron emission tomography (PET). PET is a nuclear medicine imaging technique which produces a three dimensional image of the body. PET imaging is commonly used to obtain non-invasive information about internal body structures and tissues and the function and health of such structures and tissues. PET uses a metabolically active molecule and a short-lived radioactive tracer isotope (to radiolabel the active molecule) as a diagnostic agent. The isotope decays by emitting a positron. When a PET diagnostic agent is administered to the body, it is retained or accumulates in certain tissues of interest, thereby facilitating imaging of those tissues based upon detection of gamma ray photons produced upon annihilation of positrons emitted from the radiolabeled PET diagnostic agent. Such annihilation occurs when an emitted positron collides with an electron present in the vicinity of emission, resulting in production of two 511 keV photons that are emitted in nearly opposite directions. Typically, such PET diagnostic agents are radiolabeled with (i.e., contain) one or more atoms that exhibit positron emission (such as certain isotopes of carbon, nitrogen, oxygen, fluorine, or rubidium, including ¹¹C, ¹³N, ¹⁵O, ¹⁸F, and ⁸²Rb). The effects of the emitted positrons (i.e., the subsequently emitted gamma ray photons) are detected by a detection device located outside the body which typically converts raw data into two- or three-dimensional images of the region of interest. The results are then read by a nuclear medicine physician or radiologist to interpret the results in terms of the patient's diagnosis and treatment.

Considerable effort has been invested in development of PET diagnostic agents in an effort to improve contrast between various anatomical features, such as between cancerous and non-cancerous tissues. Continued progress in medical science mandates improved options in such diagnostic capability, which in turn mandates further development of improved diagnostic agents.

Therefore, it is one object of the present invention to meet these characteristics, to overcome the drawbacks in prior methods and agents and to provide an improvement over these prior methods and agents.

### SUMMARY OF THE INVENTION

The present invention is directed to certain diagnostic agents for PET imaging and methods for using such agents exhibiting positron emission.
Accordingly, the present invention provides a diagnostic agent comprising at least one positron emitting radiolabeled halogenated xanthene selected from the group consisting of radiolabeled 4',5'-Dichlorofluorescein, radiolabeled 2',7'-Dichlorofluorescein, radiolabeled 4,5,6,7-Tetrachlorofluorescein, radiolabeled. 2',4',5',7'- Tetrachlorofluorescein, radiolabeled Diiodofluorescein, radiolabeled Erythrosin B, radiolabeled Rose Bengal, radiolabeled Mono-, Di-, or Trichloroerythrosin, radiolabeled Mono-, Di-, or Trifluoroerythrosin, radiolabeled 2',7'-Dichloro-4,5,6,7-Tetrafluorofluorescein, radiolabeled 2',4,5,6,7,7'-Hexafluorofluorescein, radiolabeled 4,5,6,7-Tetrafluorofluorescein, radiolabeled 2',4',5,5',6,7'-Hexaiodofluorescein, radiolabeled 2',4',5,5',7,'-Hexaiodofluorescein, radiolabeled 2',4',5',6,7,7'-Hexaiodofluorescein, radiolabeled 2',4',5,5',6,7,7'-Heptaiodofluorescein, radiolabeled 4-Chloro-2',4',5,5',6,7'-hexaiodofluorescein, radiolabeled 4-Chloro-2',4',5,5',7,7'-hexaiodofluorescein, radiolabeled 4-Chloro-2',4',5',6,7,7'-hexaiodofluorescein, radiolabeled 4-Chloro-2',4',5,5',6,7,7'-heptaiodofluorescein, radiolabeled 4,5-Dichloro-2',4',5',6,7,7'-hexaiodofluorescein, radiolabeled 4,6-Dichloro-2',4',5,5',7,7'-hexaiodofluorescein, and radiolabeled 4,7-Dichloro-2',4',5,5',6,7'-hexaiodofluorescein as a primary active component, wherein said agent is useful for positron emission imaging of human and animal tissue.

In a preferred embodiment, a primary component of such diagnostic agent is a halogenated xanthene that has been radiolabeled with one or more positron emitting isotopes of carbon, oxygen, fluorine, chlorines or iodine, including ¹⁰C, ¹¹C, ¹³O, ¹⁴O, ¹⁵O, ¹⁷F, ¹⁸F, ³²Cl, ³³Cl, ³⁴Cl, ¹¹⁷I, ¹¹⁸I, ¹¹⁹I, ¹²⁰I, ¹²¹I, ¹²²I, ¹²⁴I, and ¹²⁶I. In a further preferred embodiment, this radiolabeled halogenated xanthene is Rose Bengal.

These invented agents can be used in a method for imaging human or animal tissue comprising the steps of: administering a diagnostic agent to a patient, a portion of said diagnostic agent being retained in tissue of interest, said diagnostic agent emitting positrons; and imaging said tissue based on the effects of said positrons emitted from the diagnostic agent, wherein said diagnostic agent is a radiolabeled halogenated xanthene.

### BRIEF DESCRIPTION OF THE DRAWINGS

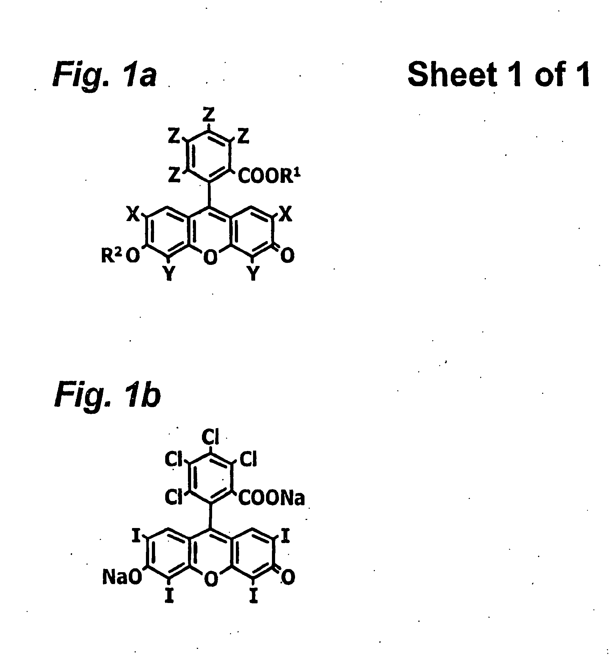

FIGURE 1a is an illustration of the chemical structure of a halogenated xanthene; and

FIGURE 1b is an illustration of the chemical structure of Rose Bengal.

### DETAILED DESCRIPTION OF THE PRESENTLY PREFERRED EMBODIMENT

The present invention is directed to certain diagnostic agents for PET imaging and methods for using such agents exhibiting positron emission. In a preferred embodiment, a primary component of such diagnostic agent is a halogenated xanthene
that has been radiolabeled with one or more positron emitting isotopes of carbon, oxygen, fluorine, chlorine, or iodine, including ¹⁰C, ¹¹C, ¹³O, ¹⁴O, ¹⁵O, ¹⁷F, ¹⁸F, ³²Cl, ³³Cl, ³⁴Cl, ¹¹⁷I, ¹¹⁸I, ¹¹⁹I, ¹²⁰I, ¹²¹I, ¹²²I, ¹²⁴I, and ¹²⁶I. In further preferred embodiment, this radiolabeled halogenated xanthene is Rose Bengal (i.e., 4,5,6,7-tetrachloro-2',4',5',7'-tetraiodofluorescein);

The halogenated xanthenes constitute a family of extremely useful agents that can be selectively and safely delivered at high concentrations to certain tissues. Certain properties of the halogenated xanthenes are described in USSN 09/184,388, filed on November 2,1998, in USSN 09/216,787, filed on December 21,1998, in USSN 09/635,276, filed on August 9, 2000, in USSN 09/799,785, filed on March 6, 2001, in USSN 09/817,448, filed on March 26, 2001, in USSN 09/900,355, filed on July 6, 2001, and in USSN 10/314,840, filed on December 9, 2002
In general, the halogenated xanthenes are characterized by a low cytotoxicity (toxicity to cells) at low concentration, a propensity for selective concentration or retention in certain tissues and cells, a high cytotoxicity upon such concentration or retention, and by chemical and physical properties that are substantially unaffected by the local chemical environment or by the attachment of functional derivatives at positions R¹ and R² described below.

The generalized chemical structure of the halogenated xanthenes is illustrated in Figure 1a, where the symbols X, Y, and Z represent various elements present at the designated positions, and the symbols R¹ and R² represent various functionalities present at the designated positions. The chemical structure of a specific example of a halogenated xanthene, Rose Bengal, is illustrated in Figure 1b. Physical properties of representative halogenated xanthenes are summarized in attached Table 1. In general, the halogenated xanthenes have the empirical formula, C₂₀HₙO₅FₐCl_{b}Br_{c}I_{d},R¹,R², where n ≥2, and a, b, c, and d are integers greater than or equal to zero. For example, the empirical formula for the disodium salt of Rose Bengal is C₂₀H₂O₅F₀Cl₄Br₀I₄,R¹,R², where R¹ and R² each represent a sodium atom, or, more simply, C₂₀H₂O₅Cl₄I₄,Na₂.

In their non-radiolabeled form, the halogenated xanthenes are useful as food dyes, biological stains, photosensitizers (i.e. agents which are used with light for photodynamic imaging or treatment as for example disclosed in USSN 09/635,276 and 09/799,785), radiosensitizers (i.e., agents that are used with applied ionizing radiation for imaging and radiation treatment as for example disclosed in USSN 09/216,787 and 09/817,448), and as chemoablative or chemotherapeutic agents (as for example disclosed in USSN 09/900,355). When labeled with certain gamma-emitting isotopes of iodine (i.e., ¹³¹I and ¹²⁵I), one of the halogenated xanthenes (Rose Bengal) has proven useful for diagnosis of hepatic function based on either measurement of differential excretion or imaging of the pattern of gamma emission from such radiolabeled molecules (for example as disclosed in Serafini et al., J. Nucl. Med. 16 (1975) 629-633).

The present inventors were part of a team that has previously discovered and disclosed that the halogenated xanthenes exhibit selective retention in certain types of tissue, especially those exhibiting cancerous or precancerous conditions (i.e., neoplasia, dysplasia, and hyperplasia), as disclosed in USSN 09/635,276, 09/799,785, 09/216,787, 09/817,448, and 09/900,355. Such retention can be useful for diagnosis (for example, using x-ray computed tomography or ultrasound imaging) and for therapy (for example, using photodynamic therapy or radiosensitization).

The present inventors have now discovered new isotopically-labeled (i.e., radiolabeled) members of the halogenated xanthene family that are capable of serving as diagnostic agents for PET imaging. More specifically, Applicants have created new halogenated xanthene compounds wherein a halogenated xanthene has been radiolabeled with one or more positron emitting isotope of carbon, oxygen, fluorine, chlorine, or iodine, including ¹⁰C, ¹¹C, ¹³O, ¹⁴O, ¹⁵O, ¹⁷F, ¹⁸F, ³²Cl, ³³Cl, ³⁴Cl, ¹¹⁷I, ¹¹⁸I, ¹¹⁹I, ¹²⁰I, ¹²¹I, ¹²²I, ¹²⁴I, and ¹²⁶I. One skilled in the art can synthesize and produce these new compounds using know chemistry of the halogenated xanthenes with the additional information provided in this application. For example, the synthesis and production of non-radiolabeled halogenated xanthenes and the chemistry of such compounds is known. Labeling with radioisotopes, such as for example the preferred radioisotopes of bromine or iodine (i.e., ⁷⁴Br, ⁷⁵Br, ⁷⁶Br, ⁷⁷Br, ⁷⁸Br, ¹¹⁷I, ¹¹⁸I, ¹¹⁹I, ¹²⁰I, ¹²¹I, ¹²²I, ¹²⁴I, ¹²⁶I, and ¹²⁸I), can be achieved by using standard halogen exchange methods, such as those taught in Serafini, *supra.* Forthese radioisotopes and the other radioisotopes listed herein, radiolabeling can be achieved using radiolabeled starting materials in the synthesis of the desired halogenated xanthene (such as for example radiolabeled resorcinol or a radiolabeled phthalic anhydride), thereby directly synthesizing the radiolabeled xanthene.

The present inventors have further discovered that a halogenated xanthene that has been radiolabeled with one or more positron emitting isotope of carbon, oxygen, fluorine, chlorine, or iodine, including ¹⁰C, ¹¹C, ¹³O, ¹⁴O, ¹⁵O, ¹⁷F, ¹⁸F, ³²Cl, ³³Cl, ³⁴Cl, ¹¹⁷I, ¹¹⁸I, ¹¹⁹I, ¹²⁰I, ¹²¹I, ¹²²I, ¹²⁴I, and ¹²⁶I, can be useful as the active substance in a diagnostic agent for PET imaging. This non-naturally-occurring, novel composition of matter will retain the desirable specificity, toxicity, and other salient pharmaceutical properties of the halogenated xanthenes, as discussed above. Some of the important physical properties of positron emitting isotopes of carbon, oxygen, fluorine, chlorine, bromine, and iodine are summarized in attached Table 2. Because positron emitting isotopes with very short half-lives may in general be impractical for use in diagnostic procedures due to logistic difficulties in their timely production and subsequent delivery to the patient, those isotopes exhibiting half-lives in excess of approximately one minute are preferred and are more useful. These preferred longer-lived positron emitting isotopes include ¹¹C, ¹⁵O, ¹⁸F, ³⁴Cl, ¹¹⁷I, ¹¹⁸I, ¹¹⁹I, ¹²⁰I, ¹²¹I, ¹²²I, ¹²⁴I, and ¹²⁶I. Because the distance traveled by the emitted positron prior to annihilation adversely affects image resolution (where traveled distance is proportional to positron energy), those longer-lived isotopes emitting lower energies are more preferred. Hence, the more preferred longer-lived, lower-energy positron emitting isotopes include ¹¹C, ¹⁸F, ¹²¹I, ¹²⁴I, and ¹²⁶I.

Thus, a diagnostic agent containing a halogenated xanthene radiolabeled with one or more of the aforementioned positron emitting elements can be used as a diagnostic agent for PET.

Hence, one preferred embodiment of the present invention is a diagnostic agent that contains, as an active ingredient at a concentration of from greater than approximately 0.001 % to less than approximately 20%, at least one halogenated xanthene radiolabeled with one or more positron emitting isotope selected from the group of ¹⁰C, ¹¹C, ¹³O, ¹⁴O, ¹⁵O, ¹⁷F, ¹⁸F, ³²Cl, ³³Cl, ³⁴Cl, ¹¹⁷I, ¹¹⁸I, ¹¹⁹I, ¹²⁰I, ¹²¹I, ¹²²I, ¹²⁴I, and ¹²⁶I (i.e. a radiolabeled halogenated xanthene). It is further preferred that this radiolabeled halogenated xanthene is radiolabeled with one or more longer-lived positron isotope selected from the group of ¹¹C, ¹⁵O, ¹⁸F, ³⁴Cl, ¹¹⁷I, ¹¹⁸I, ¹¹⁹I, ¹²⁰I, ¹²¹I, ¹²²I, ¹²⁴I, and ¹²⁶I. It is further preferred that this radiolabeled halogenated xanthene is radiolabeled with one or more longer-lived, lower-energy positron emitting isotopes selected from the group of ¹¹C, ¹⁸F, ¹²¹I, ¹²⁴I, and ¹²⁶I.

It is further preferred that this radiolabeled halogenated xanthene is radiolabeled Rose Bengal.

The radiolabeled halogenated xanthenes which are used in the diagnostic agent of the present invention are selected from the group consisting of:
radiolabeled 4',5'-Dichlorofluorescein, radiolabeled 2',7'-Dichlorofluorescein, radiolabeled 4,5,6,7-Tetrachlorofluorescein, radiolabeled 2',4',5',7'-Tetrachlorofluorescein, radiolabeled Diiodofluorescein, radiolabeled Erythrosin B, radiolabeled Rose Bengal, radiolabeled Mono-, Di-, or Trichloroerythrosin, radiolabeled Mono-, Di-, or Trifluoroerythrosin, radiolabeled 2',7'-Dichloro-4,5,6,7-Tetrafluorofluorescein, radiolabeled 2',4,5,6,7,7'-Hexafluorofluorescein, radiolabeled 4,5,6,7-Tetrafluorofluorescein, radiolabeled 2',4',5,5',6,7'-Hexaiodofluorescein, radiolabeled 2',4',5,5',7,7'-Hexaiodofluorescein, radiolabeled 2',4',5',6,7,7'-Hexaiodofluorescein, radiolabeled 2',4',5,5',6,7,7'-Heptaiodofluorescein, radiolabeled 4-Chloro-2',4',5,5',6,7'-hexaiodofluorescein, radiolabeled 4-Chloro-2',4',5,5',7,7'-hexaiodofluorescein, radiolabeled 4-Chloro-2',4',5',6,7,7'-hexaiodofluorescein, radiolabeled 4-Chloro-2',4',5,5',6,7,7'-heptaiodofluorescein, radiolabeled 4,5-Dichloro-2',4',5',6,7,7'-hexaiodofluorescein, radiolabeled 4,6-Dichloro-2',4',5,5',7,7'-hexaiodofluorescein, and radiolabeled 4,7-Dichloro-2',4',5,5',6,7'-hexaiodofluorescein radiolabeled Rose Bengal
wherein said radiolabel comprises incorporation of one or more positron emitting isotope selected from the group of ¹⁰C, ¹¹C, ¹³O, ¹⁴O, ¹⁵O, ¹⁷F, ¹⁸F, ³²Cl, ³³Cl, ³⁴Cl, ¹¹⁷I, ¹¹⁸I, ¹¹⁹I, ¹²⁰I, ¹²¹I, ¹²²I, ¹²⁴I, and ¹²⁶I.

In an alternate preferred embodiment, positron emission tomography is used to image, detect or otherwise observe the presence of a diagnostic agent that contains, at a concentration of from greater than approximately 0.001% to less than approximately 20%, at least one radiolabeled halogenated xanthene. It is further preferred that this diagnostic agent include the radiolabeled form of the halogenated xanthene Rose Bengal.

Table 1. Physical properties of some halogenated xanthenes (non-radiolabeled).

| Compound | Substitution | | | | | MW(g) |
|---|---|---|---|---|---|---|
| | X | Y | Z | R¹ | R² | |
| 4',5'-Dichlorofluorescein | Cl | H | H | Na | Na | 445 |
| 2',7'-Dichlorofluorescein | H | Cl | H | Na | Na | 445 |
| 4,5,6,7-Tetrachlorofluorescein | H | H | Cl | H | H | 470 |
| 2',4',5',7'-Tetrachlorofluorescein | Cl | Cl | H | Na | Na | 514 |
| Dibromofluorescein | Br | H | H | Na | Na | 534 |
| Solvent Red 72 | H | Br | H | H | H | 490 |
| Diiodofluorescein | I | H | H | Na | Na | 628 |
| Eosin B | NO₂ | Br | H | Na | Na | 624 |
| Eosin Y | Br | Br | H | Na | Na | 692 |
| Ethyl Eosin | Br | Br | H | C₂H₅ | K | 714 |
| Erythrosin B | I | I | H | Na | Na | 880 |
| Phloxine B | Br | Br | Cl | Na | Na | 830 |
| Rose Bengal | I | I | Cl | Na | Na | 1018 |
| 4,5,6,7-Tetrabromoerythrosin | I | I | Br | Na | Na | 1195 |

Table 2. Isotopes of relevance for PET with the halogenated xanthenes. Isotopes of bromine and ¹²⁸I are mentioned for comparative purposes.

| Element | Isotope | Positron Emission Half-Life | Emission Energy (MeV) |
|---|---|---|---|
| Carbon | ¹⁰C | 19 sec | 1.9 |
| | ¹¹C | 20 min | 1.0 |
| Oxygen | ¹³O | 0.009 sec | 6.4 |
| | ¹⁴O | 71 sec | 1.8 |
| | ¹⁵O | 124 sec | 1.7 |
| Fluorine | ¹⁷F | 66 sec | 1.7 |
| | ¹⁸F | 110 min | 0.6 |
| Chlorine | ³²Cl | 0.3 sec | 9.5 |
| | ³³Cl | 2.5 sec | 4.5 |
| | ³⁴Cl | 32 min | 2.5 |
| Bromine | ⁷⁴Br | 36 min | 4.7 |
| | ⁷⁵Br | 1.7 hours | 0.3 |
| | ⁷⁶Br | 16.1 hours | 1.2-3.6 |
| | ⁷⁷Br | 57 hours | 0.4 |
| | ⁷⁸Br | 6.4 min | 1.9 |
| Iodine | ¹¹⁷I | 7 min | |
| | ¹¹⁸I | 14 min | 5.5 |
| | ¹¹⁹I | 19 min | |
| | ¹²⁰I | 1.3 hours | 2.1-4.0 |
| | ¹²¹I | 2.1 hours | 1.2 |
| | ¹²²I | 3.5 min | 1.8-3.1 |
| | ¹²⁴I | 4.2 days | 0.8-2.1 |
| | ¹²⁶I | 13 days | 1.1 |
| | ¹²⁸I | 25 min | |

## Claims

1. A diagnostic agent comprising at least one positron emitting radiolabeled halogenated xanthene selected from the group consisting of radiolabeled 4',5'-Dichlorofluorescein, radiolabeled 2',7'-Dichlorofluorescein, radiolabeled 4,5,6,7-Tetrachlorofluorescein, radiolabeled 2',4',5',7'-Tetrachlorofluorescein, radiolabeled Diiodofluorescein, radiolabeled Erythrosin B, radiolabeled Rose Bengal, radiolabeled Mono-, Di-, or Trichloroerythrosin, radiolabeled Mono-, Di-, or Trifluoroerythrosin, radiolabeled 2',7'-Dichloro-4,5,6,7-Tetrafluorofluorescein, radiolabeled 2',4,5,6,7,7'-Hexafluorofluorescein, radiolabeled 4,5,6,7-Tetrafluorofluorescein, radiolabeled 2',4',5,5',6,7'-Hexaiodofluorescein, radiolabeled 2',4',5,5',7,7'-Hexaiodofluorescein, radiolabeled 2',4',5',6,7,7'-Hexaiodofluorescein, radiolabeled 2',4',5,5',6,7,7'-Heptaiodofluorescein, radiolabeled 4-Chloro-2',4',5,5',6,7'-hexaiodofluorescein, radiolabeled 4-Chloro-2',4',5,5',7,7'-hexaiodofluorescein, radiolabeled 4-Chloro-2',4',S',6,7,7'-hexaiodofluorescein, radiolabeled 4-Chloro-2',4',5,5',6,7,7'-heptaiodofluorescein, radiolabeled 4,5-Dichloro-2',4',5',6,7,7'-hexaiodofluorescein, radiolabeled 4,6-Dichloro-2',4',5,5',7,7'-hexaiodofluorescein, and radiolabeled 4,7-Dichloro-2',4',5,5',6,7'-hexaiodofluorescein as a primary active component, wherein said agent is useful for positron emission imaging of human and animal tissue.

2. A diagnostic agent according to claim 1, wherein said halogenated xanthene has been radiolabeled with at least one positron emitting isotope selected from the group of elements consisting of carbon, oxygen, fluorine, chlorine, and iodine.

3. A diagnostic agent according to claim 1, wherein said radiolabeled halogenated xanthene is Rose Bengal.

4. A method for imaging human or animal tissue comprising the steps of:
administering a diagnostic agent to a patient, a portion of said diagnostic agent being retained in tissue of interest, said diagnostic agent emitting positrons; and
imaging said tissue based on the effects of said positrons emitted from the diagnostic agent, wherein said diagnostic agent comprises a positron emitting radiolabeled halogenated xanthene selected from the group consisting of radiolabeled 4',5'-Dichlorofluorescein, radiolabeled 2',7'-Dichlorofluorescein, radiolabeled 4,5,6,7-Tetrachlorofluorescein, radiolabeled 2',4',5',7'-Tetrachlorofluorescein, radiolabeled Diiodofluorescein, radiolabeled Erythrosin B, radiolabeled Rose Bengal, radiolabeled Mono-, Di-, or Trichloroerythrosin, radiolabeled Mono-, Di-, or Trifluoroerythrosin, radiolabeled 2',T-Dichloro-4,5,6,7-Tetrafluorofluorescein, radiolabeled 2',4,5,6,7,7'-Hexafluorofluorescein, radiolabeled 4,5,6,7-Tetrafluorofluorescein, radiolabeled 2',4',5,5',6,7'-Hexaiodofluorescein, radiolabeled 2',4',5,5',7,7'-Hexaiodofluorescein, radiolabeled 2',4',5',6,7,7'-Hexaiodofluorescein, radiolabeled 2',4',5,5',6,7,7'-Heptaiodofluorescein, radiolabeled 4-Chloro-2',4',5,5',6,7'-hexaiodofluorescein, radiolabeled 4-Chloro-2',4',5,5',7,7'-hexaiodofluorescein, radiolabeled 4-Chloro-2',4',5',6,7,7'-hexaiodofluorescein, radiolabeled 4-Chloro-2',4',5,5',6,7,7'-heptaiodofluorescein, radiolabeled 4,5-Dichloro-2',4',5',6,7,7'-hexaiodofluorescein, radiolabeled 4,6-Dichloro-2',4',5,5',7,7'-hexaiodofluorescein, and radiolabeled 4,7-Dichloro-2',4',5,5',6,7'-hexaiodofluorescein.

5. The method according to claim 4 wherein said radiolabeled halogenated xanthene is radiolabeled Rose Bengal.

6. The diagnostic agent or method according to any preceding claim wherein said radiolabel comprises incorporation, into said halogenated xanthene, of one or more positron emitting isotope selected from the group consisting of ¹⁰C, ¹¹C, ¹³O ¹⁴O, ¹⁵O, ¹⁷F, ¹⁸F, ³²Cl, ³³Cl, ³⁴Cl, ¹¹⁷I, ¹¹⁸I, ¹¹⁹I, ¹²⁰I, ¹²¹I, ¹²²I, ¹²⁴I and ¹²⁶I.

7. The diagnostic agent or method according to any preceding claim wherein said radiolabeled halogenated xanthene is present in a concentration of greater than about 0.001% to less than about 20%.

8. A positron emitting halogenated xanthene selected from the group consisting of radiolabeled 4',5'-Dichlorofluorescein, radiolabeled 2',7'-Dichlorofluorescein, radiolabeled 4,5,6,7-Tetrachlorofluorescein, radiolabeled 2',4',5',7'- Tetrachlorofluorescein, radiolabeled Diiodofluorescein, radiolabeled Erythrosin B, radiolabeled Rose Bengal, radiolabeled Mono-, Di-, or Trichloroerythrosin, radiolabeled Mono-, Di-, or Trifluoroerythrosin, radiolabeled 2',7'-Dichloro-4,5,6,7-Tetrafluorofluorescein, radiolabeled 2',4,5,6,7,7'-Hexafluorofluorescein, radiolabeled 4,5,6,7-Tetrafluorofluorescein, radiolabeled 2',4',5,5',6,7'-Hexaiodofluorescein, radiolabeled 2',4',5,5',7,7'-Hexaiodofluorescein, radiolabeled 2',4',5',6,7,7'-Hexaiodofluorescein, radiolabeled 2',4',5,5',6,7,7'-Heptaiodofluorescein, radiolabeled 4-Chloro-2',4',5,5',6,7'-hexaiodofluorescein, radiolabeled 4-Chloro-2',4',5,5',7,7'-hexaiodofluorescein, radiolabeled 4-Chloro-2',4',5',6,7,7'-hexaiodofluorescein, radiolabeled 4-Chloro-2',4',5,5',6,7,7'-heptaiodofluorescein, radiolabeled 4,5-Dichloro-2',4',5',6,7,7'-hexaiodofluorescein, radiolabeled 4,6-Dichloro-2',4',5,5',7,7'-hexaiodofluorescein, and radiolabeled 4,7-Dichloro-2',4',5,5',6,7'-hexaiodofluorescein, wherein said halogenated xanthene has been radiolabeled with at least one positron emitting isotope selected from the group consisting of ¹⁰C, ¹¹C, ¹³O, ¹⁴O, ¹⁵O, ¹⁷F, ¹⁸F, ³²Cl, ³³Cl, ³⁴Cl, ¹¹⁷I, ¹¹⁸I, ¹¹⁹I, ¹²⁰I, ¹²¹I, ¹²²I, ¹²⁴I, and ¹²⁶I.

## Patentansprüche

1. Ein Diagnosemittel, das mindestens ein positronenemittierendes radioaktiv markiertes halogeniertes Xanthen umfasst, das ausgewählt ist aus der Gruppe umfassend radioaktiv markiertes 4',5'-Dichlorfluorescein, radioaktiv markiertes 2',7'-Dichlorfluorescein, radioaktiv markiertes 4,5,6,7-Tetrachlorfluorescein, radioaktiv markiertes 2',4',5',7'-Tetrachlorfluorescein, radioaktiv markiertes Diiodfluorescein, radioaktiv markiertes Erythrosin B, radioaktiv markiertes Bengalrosa, radioaktiv markiertes Mono-, Di- oder Trichlorerythrosin, radioaktiv markiertes Mono-, Di- oder Trifluorerythrosin, radioaktiv markiertes 2',7'-Dichlor-4,5,6,7-Tetrafluorfluorescein, radioaktiv markiertes 2',4,5,6,7,7'-Hexafluorfluorescein, radioaktiv markiertes 4,5,6,7-Tetrafluorfluorescein, radioaktiv markiertes 2',4',5,5',6,7'-Hexaiodfluorescein, radioaktiv markiertes 2',4',5,5',7,7'-Hexaiodfluorescein, radioaktiv markiertes 2',4',5',6,7,7'-Hexaiodfluorescein, radioaktiv markiertes 2',4',5,5',6,7,7'-Heptaiodfluorescein, radioaktiv markiertes 4-Chlor-2',4',5,5',6,7'-hexaiodfluorescein, radioaktiv markiertes 4-Chlor-2',4',5,5',7,7'-hexaiodfluorescein, radioaktiv markiertes 4-Chlor-2',4',5',6,7,7'-hexaiodfluorescein, radioaktiv markiertes 4-Chlor-2',4',5,5',6,7,7'-heptaiodfluorescein, radioaktiv markiertes 4,5-Dichlor-2',4',5',6,7,7'-hexaiodfluorescein, radioaktiv markiertes 4,6-Dichlor-2',4',5,5',7,7'-hexaiodfluorescein und radioaktiv markiertes 4,7-Dichlor-2',4',5,5',6,7'-hexaiodfluorescein als eine primäre aktive Komponente, worin das Diagnosemittel von Nutzen für die Positronenemissionsbildgebung von menschlichem und tierischem Gewebe ist.

2. Ein Diagnosemittel gemäß Anspruch 1, worin das halogenierte Xanthen mit mindestens einem positronenemittierenden Isotop radioaktiv markiert wurde, das ausgewählt ist aus der Gruppe, die Kohlenstoff, Sauerstoff, Fluor, Chlor und Iod umfasst.

3. Ein Diagnosemittel gemäß Anspruch 1, worin das radioaktiv markierte halogenierte Xanthen Bengalrosa ist.

4. Ein Verfahren zur Bildgebung von menschlichem oder tierischem Gewebe, das folgende Schritte umfasst:
Verabreichung eines Diagnosemittels an einen Patienten, wobei ein Teil des Diagnosemittels in dem interessierenden Gewebe zurückgehalten wird, wobei das Diagnosemittel Positronen emittiert; und
Bildgebung des Gewebes auf der Grundlage der Wirkungen der Positronen, die von dem Diagnosemittel emittiert werden, worin das Diagnosemittel ein positronenemittierendes halogeniertes Xanthen umfasst, das aus der Gruppe ausgewählt ist umfassend radioaktiv markiertes 4',5'-Dichlorfluorescein, radioaktiv markiertes 2',7'-Dichlorfluorescein, radioaktiv markiertes 4,5,6,7-Tetrachlorfluorescein, radioaktiv markiertes 2',4',5',7'-Tetrachlorfluorescein, radioaktiv markiertes Diiodfluorescein, radioaktiv markiertes Erythrosin B, radioaktiv markiertes Bengalrosa, radioaktiv markiertes Mono-, Di- oder Trichlorerythrosin, radioaktiv markiertes Mono-, Di- oder Trifluorerythrosin, radioaktiv markiertes 2',7'-Dichlor-4,5,6,7-Tetrafluorfluorescein, radioaktiv markiertes 2',4,5,6,7,7'-Hexafluorfluorescein, radioaktiv markiertes 4,5,6,7-Tetrafluorfluorescein, radioaktiv markiertes 2',4',5,5',6,7'-Hexaiodfluorescein, radioaktiv markiertes 2',4',5,5',7,7'-Hexaiodfluorescein, radioaktiv markiertes 2',4',5',6,7,7'-Hexaiodfluorescein, radioaktiv markiertes 2',4',5,5',6,7,7'-Heptaiodfluorescein, radioaktiv markiertes 4-Chlor-2',4',5,5',6,7'-hexaiodfluorescein, radioaktiv markiertes 4-Chlor-2',4',5,5',7,7'-hexaiodfluorescein, radioaktiv markiertes 4-Chlor-2',4',5',6,7,7'-hexaiodfluorescein, radioaktiv markiertes 4-Chlor-2',4',5,5',6,7,7'-heptaiodfluorescein, radioaktiv markiertes 4,5-Dichlor-2',4',5',6,7,7'-hexaiodfluorescein, radioaktiv markiertes 4,6-Dichlor-2',4',5,5',7,7'-hexaiodfluorescein und radioaktiv markiertes 4,7-Dichlor-2',4',5,5',6,7'-hexaiodfluorescein.

5. Das Verfahren gemäß Anspruch 4, worin das radioaktiv markierte halogenierte Xanthen radioaktiv markiertes Bengalrosa ist.

6. Das Diagnosemittel oder Verfahren gemäß einem der vorherigen Ansprüche, worin die radioaktive Markierung die Inkorporation in das halogenierte Xanthen von einem oder mehr positronenemittierenden Isotopen umfasst, die ausgewählt sind aus der Gruppe umfassend ¹⁰C, ¹¹C, ¹³O, ¹⁴O, ¹⁵O, ¹⁷F, ¹⁸F, ³²Cl, ³³Cl, ³⁴Cl, ¹¹⁷I, ¹¹⁸I, ¹¹⁹I, ¹²⁰I, ¹²¹I, ¹²²I, ¹²⁴I und ¹²⁶I.

7. Das Diagnosemittel oder Verfahren gemäß einem der vorherigen Ansprüche, worin das radioaktiv markierte halogenierte Xanthen in einer Konzentration von größer als etwa 0,001% bis kleiner als etwa 20% vorliegt.

8. Ein positronenemittierendes radioaktiv markiertes halogeniertes Xanthen, das ausgewählt ist aus der Gruppe umfassend radioaktiv markiertes 4',5'-Dichlorfluorescein, radioaktiv markiertes 2',7'-Dichlorfluorescein, radioaktiv markiertes 4,5,6,7-Tetrachlorfluorescein, radioaktiv markiertes 2',4',5',7'-Tetrachlorfluorescein, radioaktiv markiertes Diiodfluorescein, radioaktiv markiertes Erythrosin B, radioaktiv markiertes Bengalrosa, radioaktiv markiertes Mono-, Di- oder Trichlorerythrosin, radioaktiv markiertes Mono-, Di- oder Trifluorerythrosin, radioaktiv markiertes 2',7'-Dichlor-4,5,6,7-Tetrafluorfluorescein, radioaktiv markiertes 2',4,5,6,7,7'-Hexafluorfluorescein, radioaktiv markiertes 4,5,6,7-Tetrafluorfluorescein, radioaktiv markiertes 2',4',5,5',6,7'-Hexaiodfluorescein, radioaktiv markiertes 2',4',5,5',7,7'-Hexaiodfluorescein, radioaktiv markiertes 2',4',5',6,7,7'-Hexaiodfluorescein, radioaktiv markiertes 2',4',5,5',6,7,7'-Heptaiodfluorescein, radioaktiv markiertes 4-Chlor-2',4',5,5',6,7'-hexaiodfluorescein, radioaktiv markiertes 4-Chlor-2',4',5,5',7,7'-hexaiodfluorescein, radioaktiv markiertes 4-Chlor-2',4',5',6,7,7'-hexaiodfluorescein, radioaktiv markiertes 4-Chlor-2',4',5,5',6,7,7'-heptaiodfluorescein, radioaktiv markiertes 4,5-Dichlor-2',4',5',6,7,7'-hexaiodfluorescein, radioaktiv markiertes 4,6-Dichlor-2',4',5,5',7,7'-hexaiodfluorescein und radioaktiv markiertes 4,7-Dichlor-2',4',5,5',6,7'-hexaiodfluorescein, worin das halogenierte Xanthen mit mindestens einem positronenemittierenden Isotop radioaktiv markiert ist, das ausgewählt ist aus der Gruppe umfassend ¹⁰C, ¹¹C, ¹³O, ¹⁴O, ¹⁵O, ¹⁷F, ¹⁸F, ³²Cl, ³³Cl, ³⁴Cl, ¹¹⁷I, ¹¹⁸I, ¹¹⁹I, ¹²⁰I, ¹²¹I, ¹²²I, ¹²⁴I und ¹²⁶I.

## Revendications

1. Agent diagnostique comprenant au moins un xanthène halogéné radiomarqué émettant des positrons sélectionné parmi le groupe consistant en la 4',5'-dichlorofluorescéine radiomarquée, la 2',7'-dichlorofluorescéine radiomarquée, la 4,5,6,7-tétrachlorofluorescéine radiomarquée, la 2',4',5',7'-tétrachlorofluorescéine radiomarquée, la diiodofuorescéine radiomarquée, l'érythrosine B radiomarquée, le rose bengale radiomarqué, la mono-, di- ou trichloroérythrosine radiomarquée, la mono-, di- ou trifluororoérythrosine radiomarquée, la 2',7'-dichloro-4,5,6,7-tétrafluororofluorescéine radiomarquée, la 2',4,5,6,7,7'-hexafluorofluorescéine radiomarquée, la 4,5,6,7-tétrafluorofluorescéine radiomarquée, la 2',4',5,5'6,7'-hexaiodofluorescéine radiomarquée, la 2',4',5,5',7,7'-hexaiodofluorescéine radiomarquée, , la 2',4',5',6,7,7'-hexaiodofluorescéine radiomarquée, la 2',4',5,5'6,7,7'-heptaiodofluorescéine radiomarquée, la 4-chloro-2',4',5,5',6,7'-hexaiodofluorescéine radiomarquée, la 4-chloro-2',4',5,5',7,7'-hexaiodofluorescéine radiomarquée, la 4-chloro-2',4',5',6,7,7'-hexaiodofluorescéine radiomarquée, la 4-chloro-2',4',5,5',6,7,7'-heptaiodofluorescéine radiomarquée, la 4,5-dichloro-2',4',5',6,7,7'-hexaiodofluorescéine radiomarquée, la 4,6-dichloro-2',4',5,5',7,7'-hexaiodofluorescéine radiomarquée, et la 4,7-dichloro-2',4',5,5',6, 7'-hexaiodofluorescéine radiomarquée comme composant actif primaire, ledit agent étant utile pour l'imagerie par émission de positrons de tissus humains et animaux.

2. Agent diagnostique selon la revendication 1, ledit xanthène halogéné étant radiomarqué avec au moins un isotope émettant des positrons sélectionné dans le groupe d'éléments consistant en carbone, oxygène, fluor, chlore et iode.

3. Agent diagnostique selon la revendication 1, ledit xanthène halogéné étant le rose bengale.

4. Procédé pour l'imagerie de tissus humains ou animaux comprenant les étapes suivantes :
administration d'un agent diagnostique à un patient, une partie dudit agent diagnostique étant retenue dans le tissu étudié, ledit agent diagnostique émettant des positrons ; et
imagerie dudit tissu basée sur les effets desdits positrons émis depuis l'agent diagnostique, ledit agent diagnostique comprenant un xanthène halogéné radiomarqué émettant des positrons sélectionné parmi le groupe consistant en la 4',5'-dichlorofluorescéine radiomarquée, la 2',7'-dichlorofluorescéine radiomarquée, la 4,5,6,7-tétrachlorofluorescéine radiomarquée, la 2',4',5',7'-tétrachlorofluorescéine radiomarquée, la diiodofuorescéine radiomarquée, l'érythrosine B radiomarquée, le rose bengale radiomarqué, la mono-, di- ou trichloroérythrosine radiomarquée, la mono-, di- ou trifluororoérythrosine radiomarquée, la 2',7'-dichloro-4,5,6,7-tétrafluororofluorescéine radiomarquée, la 2',4,5,6,7,7'-hexafluorofluorescéine radiomarquée, la 4,5,6,7-tétrafluorofluorescéine radiomarquée, la 2',4',5,5'6,7'-hexaiodofluorescéine radiomarquée, la 2',4',5,5',7,7'-hexaiodofluorescéine radiomarquée, , la 2',4',5',6,7,7'-hexaiodofluorescéine radiomarquée, la 2',4',5,5'6,7,7'-heptaiodofluorescéine radiomarquée, la 4-chloro-2',4',5,5',6,7'-hexaiodofluorescéine radiomarquée, la 4-chloro-2',4',5,5',7,7'-hexaiodofluorescéine radiomarquée, la 4-chloro-2',4',5',6,7,7'-hexaiodofluorescéine radiomarquée, la 4-chloro-2',4',5,5',6,7,7'-heptaiodofluorescéine radiomarquée, la 4,5-dichloro-2',4',5',6,7,7'-hexaiodofluorescéine radiomarquée, la 4,6-dichloro-2',4',5,5',7,7'-hexaiodofluorescéine radiomarquée, et la 4,7-dichloro-2',4',5,5',6, 7'-hexaiodofluorescéine radiomarquée.

5. Procédé selon la revendication 4, ledit xanthène halogéné radiomarqué étant le rose bengale.

6. Agent diagnostique ou méthode selon n'importe quelle revendication précédente, ledit radiomarqueur comprenant l'incorporation, dans ledit xanthène halogéné, d'un ou plusieurs isotopes émetteurs de positrons sélectionnés parmi le groupe consistant en ¹⁰C, ¹¹C, ¹³O, ¹⁴0, ¹⁵O, ¹⁷Fe, ¹⁸F, ³²Cl, ³³Cl, ³⁴CL, ¹¹⁷I, ¹¹⁸I, ¹¹⁹I, ¹²⁰I, ¹²¹I, ¹²²I, ¹²⁴I et ¹²⁶I.

7. Agent diagnostique ou méthode selon n'importe quelle revendication précédente, ledit xanthène halogéné radiomarqué étant présent à une concentration de plus d'environ 0,001 % à moins d'environ 20 %.

8. Xanthène halogéné émettant des positrons sélectionné parmi le groupe consistant en la 4',5'-dichlorofluorescéine radiomarquée, la 2',7'-dichlorofluorescéine radiomarquée, la 4,5,6,7-tétrachlorofluorescéine radiomarquée, la 2',4',5',7'-tétrachlorofluorescéine radiomarquée, la diiodofuorescéine radiomarquée, l'érythrosine B radiomarquée, le rose bengale radiomarqué, la mono-, di- ou trichloroérythrosine radiomarquée, la mono-, di- ou trifluororoérythrosine radiomarquée, la 2',7'-dichloro-4,5,6,7-tétrafluororofluorescéine radiomarquée, la 2',4,5,6,7,7'-hexafluorofluorescéine radiomarquée, la 4,5,6,7-tétrafluorofluorescéine radiomarquée, la 2',4',5,5'6,7'-hexaiodofluorescéine radiomarquée, la 2',4',5,5',7,7'-hexaiodofluorescéine radiomarquée, , la 2',4',5',6,7,7'-hexaiodofluorescéine radiomarquée, la 2',4',5,5'6,7,7'-heptaiodofluorescéine radiomarquée, la 4-chloro-2',4',5,5',6,7'-hexaiodofluorescéine radiomarquée, la 4-chloro-2',4',5,5',7,7'-hexaiodofluorescéine radiomarquée, la 4-chloro-2',4',5',6,7,7'-hexaiodofluorescéine radiomarquée, la 4-chloro-2',4',5,5',6,7,7'-heptaiodofluorescéine radiomarquée, la 4,5-dichloro-2',4',5',6,7,7'-hexaiodofluorescéine radiomarquée, la 4,6-dichloro-2',4',5,5',7,7'-hexaiodofluorescéine radiomarquée, et la 4,7-dichloro-2',4',5,5',6, 7'-hexaiodofluorescéine radiomarquée, ledit xanthène halogéné ayant été radiomarqué avec au moins un isotope émetteur de positrons sélectionné parmi le groupe consistant en ¹⁰C, ¹¹C, ¹³O, ¹⁴0, ¹⁵O, ¹⁷Fe, ¹⁸F, ³²Cl, ³³Cl, ³⁴CL, ¹¹⁷I, ¹¹⁸I, ¹¹⁹I, ¹²⁰I, ¹²¹I, ¹²²I, ¹²⁴I, et ¹²⁶I.
